(19) **Europäisches Patentamt / European Patent Office / Office européen des brevets**

(11) **EP 3 549 623 B1**

(12) **EUROPEAN PATENT SPECIFICATION**

(45) Date of publication and mention of the grant of the patent:
**16.08.2023 Bulletin 2023/33**

(21) Application number: **17875547.6**

(22) Date of filing: **24.11.2017**

(51) International Patent Classification (IPC):
**B01J 20/24** (2006.01)   **B01J 20/30** (2006.01)
**B01J 20/28** (2006.01)   **A61M 1/02** (2006.01)
**A61K 35/15** (2015.01)

(52) Cooperative Patent Classification (CPC):
**B01J 20/24; A61M 1/02; B01J 20/28;
B01J 20/28007; B01J 20/28033; B01J 20/28059;
B01J 20/28061; B01J 20/28085; B01J 20/3085;**
A61K 35/15

(86) International application number:
**PCT/JP2017/042138**

(87) International publication number:
**WO 2018/101156 (07.06.2018 Gazette 2018/23)**

(54) **USE OF A BLOOD COMPONENT SELECTIVE ADSORPTION FILTERING MEDIUM AND BLOOD FILTER**

VERWENDUNG EINES BLUTKOMPONENTENSELEKTIVEN ADSORPTIONSFILTERMEDIUM UND BLUTFILTER

UTILISATION D'UN MILIEU FILTRANT D'ADSORPTION SÉLECTIVE DE COMPOSANT SANGUIN ET FILTRE SANGUIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.11.2016 JP 2016231356**

(43) Date of publication of application:
**09.10.2019 Bulletin 2019/41**

(73) Proprietor: **FUJIFILM Corporation Tokyo 106-8620 (JP)**

(72) Inventors:
• **KAMINAGA Kuniyuki
  Ashigarakami-gun
  Kanagawa 258-8577 (JP)**
• **TAKEGAMI Ryuta
  Ashigarakami-gun
  Kanagawa 258-8577 (JP)**

(74) Representative: **Hoffmann Eitle
Patent- und Rechtsanwälte PartmbB
Arabellastraße 30
81925 München (DE)**

(56) References cited:
**JP-A- H0 624 995      JP-A- 2001 198 215
JP-A- 2009 226 840      JP-A- 2016 053 232**

• **Daicel Com: "Technical information Production method for cellulose acetate Production items of cellulose acetate Solvent for and solubility of cellulose acetate Solution viscosity of cellulose acetate Compatibility of cellulose acetate Precautions in handling cellulose acetate Safety assessment", Technical Information, 1 January 2019 (2019-01-01), pages 1-5, XP055637918, Retrieved from the Internet: URL:https://www.daicel.com/cell_ac/en/cellulose/detail.html [retrieved on 2019-10-31]**

**Description**

**BACKGROUND OF THE INVENTION**

1. Field of the Invention

The present invention relates to the non-therapeutic use of a filtering medium for selective adsorption of blood components, and a blood filter comprising the filter medium.

2. Description of the Related Art

In the field of blood transfusion, whole blood preparations, erythrocyte preparations, platelet preparations, plasma preparations, and the like are used depending on the use of a blood recipient. In recent years, a so-called leukocyte removal blood transfusion, in which leukocytes contained in such blood preparations are removed, and then the blood preparations are transfused, has become widespread.

For example, JP3172542B discloses a "filter material for trapping leukocytes, which is characterized in that a porous body filter material of a three-dimensional network continuous structure, which is formed from at least one polymer material, and which has an average pore diameter of 1 to 60 $\mu$m, a specific surface area of 0.5 to 10 m$^2$/g, a porosity of 30% to 95%, a bubble point of 0.08 to 0.30 kg/cm$^2$, a wall thickness of 0.1 to 9.0 mm, and a surface porosity of 6% to 90%, was fixed to at least one surface of a fiber filter material made of nonwoven fabric" ([Claim 1]).

In addition, WO2005/120600A discloses "a filter for removing leukocytes characterized by containing a porous filter material in which an osmotic coefficient (kx) in a direction in which the filter is perpendicular to a filtration surface is $0.5 \times 10^{-12}$ m$^2$ or more and $2.0 \times 10^{-12}$ m$^2$ or less, and a ratio (ky/kx) of an osmotic coefficient (ky) in a direction in which the filter is parallel to a filtration surface, to kx is 0.5 or more and 1.5 or less, in the filter for removing leukocytes from a leukocyte-containing liquid, in which a container having a liquid inlet and an outlet is filled with the porous filter material" ([Claim 4]); discloses a filter material formed from a fiber aggregate such as a nonwoven fabric, or from a porous body as the porous filter material; and discloses a material such as cellulose acetate as a material of the porous body (<0030>).

JP 2016 053232 A discloses a method for producing nanofibers from a cellulose acylate solution.

JP 2001 198215 A relates to a leukocyte selective removal filter wherein a cellulosic polymer is provided at least on the surface of a filter base medium and the content of the cellulosic polymer in the filter is 0.1 mg/g to 200 mg/g.

JP H06 24995 A is directed to a leukocyte capturing material comprising a porous element having an average pore diameter of 1 to 25 $\mu$m and a total pore volume of 0.40 to 0.95 ml / ml (porous element), wherein the volume of the pore portion of 1 to 30 $\mu$m of the porous element is 90% or more of the total pore volume.

**SUMMARY OF THE INVENTION**

The inventors of the present invention have conducted examinations on the known materials of the related art, which are disclosed in JP3172542B, WO2005/120600A, and the like as a filtering medium which selectively adsorbs blood components such as a leukocyte, and have elucidated that, depending on the type of a polymer material to be used, a removal (trapping) rate of leukocytes may be remarkably decreased in some cases according to conditions of sterilization treatment applied to the filtering medium.

Accordingly, an object of the present invention is to provide a filtering medium for selective adsorption of blood components, which is excellent in selective removal of blood components such as a leukocyte; and a blood filter formed of the filtering medium.

As a result of intensive studies to achieve the above object, the inventors of the present invention have found that a filtering medium containing cellulose acylate, and having a glass transition temperature of 126°C or higher, and an average through-hole diameter and a specific surface area within a predetermined range, is excellent in selective removal of blood components such as a leukocyte, and therefore have completed the present invention.

That is, the inventors have found that the above-described object can be achieved by the following configuration.

[1] Non-therapeutic use of a filtering medium for selective adsorption of blood components, which contains a cellulose acylate, and the filtering medium has a glass transition temperature of 126°C or higher, an average through-hole diameter of 0.1 to 50 $\mu$m, and a specific surface area of 1.0 to 100 m$^2$/g as specified in claim 1.

[2] Use according to [1], in which a substitution degree of an acyl group included in the cellulose acylate is within a range of 1.00 to 2.90 as specified in claim 2.

[3] Use according to [1] or [2], in which the acyl group included in the cellulose acylate has 4 or less carbon atoms.

[4] Use according to any one of [1] to [3], in which the acyl group included in the cellulose acylate includes at least an acetyl group.

[5] Use according to any one of [1] to [4], in which the cellulose acylate constitutes at least a part of a surface of a member that comes into contact with blood.

[6] Use according to any one of [1] to [5], wherein the filtering medium is a nonwoven fabric or a porous membrane.

[7] Use according to any one of [1] to [6], wherein the cellulose acylate includes two or more acyl groups selected from the group consisting of an acetyl group, a propionyl group, and a butyryl group.

[8] Use according to any one of [1] to [7], wherein the average through-hole diameter is 5 to 15 $\mu$m and the specific surface area is 3 to 50 m$^2$/g.

[9] Use according to any one of [1] to [8], wherein the filtering medium is a nonwoven fabric made of fibers having an average fiber diameter of 1 nm to 5 $\mu$m and an average fiber length of 1 mm to 1 m as specified in claim 9.

[10] Use according to any one of [1] to [9], wherein the blood component is a blood cell component.

[11] Use according to any one of [1] to [10], wherein the blood component is a leukocyte component.

[12] A blood filter which is filled with the filtering medium for selective adsorption of blood components as defined in any one of [1] to [11].

According to the present invention, it is possible to provide a filtering medium for selective adsorption of blood components, which is excellent in selective removal of blood components such as a leukocyte; and a blood filter formed of the filtering medium.

## DESCRIPTION OF THE PREFERRED EMBODIMENTS

Hereinafter, the invention will be described in detail.

The explanation of the configuration requirements described below is based on representative embodiments of the invention; however, the invention is not intended to be limited to such embodiments.

According to the present specification, a numerical value range indicated using "to" means a range including the numerical values described before and after "to" as the lower limit and the upper limit.

[Filtering medium for selective adsorption of blood components]

A filtering medium for selective adsorption of blood components used in the embodiment of the present invention (hereinafter abbreviated simply as the "filtering medium for adsorption of the embodiment of the present invention") contains a cellulose acylate.

In addition, the filtering medium for adsorption of the embodiment of the present invention has a glass transition temperature of 126°C or higher, an average through-hole diameter of 0.1 to 50 $\mu$m, and a specific surface area of 1.0 to 100 m$^2$/g.

Here, the "cellulose acylate" refers to a cellulose ester in which some or all of the hydrogen atoms that constitute hydroxyl groups of cellulose, that is, free hydroxyl groups existing at the 2-position, 3-position, and 6-position of a $\beta$-1,4-bonded glucose unit, have been substituted with acyl groups.

As described above, because the filtering medium for adsorption of the embodiment of the present invention contains a cellulose acylate, and has the glass transition temperature of 126°C or higher, the average through-hole diameter of 0.1 to 50 $\mu$m, and the specific surface area of 1.0 to 100 m$^2$/g, the filtering medium is excellent in selective removal of blood components such as a leukocyte.

The reason why such effects are exhibited is not clearly known in detail; however, the inventors of the present invention speculate the reason as follows.

In other words, in the present invention, it is considered that a degree of freedom in designing an average through-hole diameter and a specific surface area increases since a cellulose acylate can also be adsorbed onto blood components (particularly a leukocyte) directly or through a protein; or a glass transition temperature of the filtering medium is high since a cellulose acylate is contained, and therefore defects such as clogging can be suppressed even in a case where the filtering medium is exposed to a high temperature state by sterilization treatment or the like.

In the present invention, the glass transition temperature of the filtering medium for selective adsorption of blood components is 126°C or higher, but is preferably 135°C or higher, more preferably 145°C or higher, and even more preferably 150°C to 300°C, from the reason of improving a selective removal rate of blood components (particularly a leukocyte).

The "glass transition temperature" can be obtained as a temperature at an intersection of a straight line obtained by extending a base line on a low temperature side to a high temperature side, and a tangential line drawn at an inflection point of a curve of a stepwise changing portion of glass transition, in a curve derived from the glass transition of a cellulose acylate obtained in a case of measurement at a rate of temperature increase of 10°C/min using a Differential Scanning Calorimeter (DSC). The measurement is carried out in two cycles, and a temperature obtained from a curve of a second cycle by the above-mentioned method is referred to as a glass transition temperature. In this case, in a first cycle, at a rate of temperature increase of 10°C/min, the filtering medium is heated to a temperature at least 30°C higher than that at the time of the completion of the glass transition in a case of a non-crystalline state, and the filtering medium is heated to a temperature at least 30°C higher than that at the time of the completion of a melting peak in a case of a crystalline state. The filtering medium is kept at each temperature for 10 minutes, and then rapidly cooled to a temperature about 50°C below the glass transition temperature. In addition, the second cycle is performed following the first cycle, and a curve obtained by heating at a rate of temperature increase of 10°C/min is used for calculation of a glass transition point.

Furthermore, the "glass transition temperature" can be appropriately adjusted depending on a substitution degree of cellulose acylate contained in the filtering medium for adsorption of the embodiment of the present invention, the type of substituents, and the like.

The filtering medium for selective adsorption of blood components of the embodiment of the present invention has not only an effect of removing blood components by adsorption but also a separation and removal effect due to a size difference between different blood cell components. According to these two effects, different kinds of blood components can be effectively separated. In the present invention, an optimal size as an average through-hole diameter of the filtering medium can be appropriately selected for a blood component to be separated, but for selective separation, the average through-hole diameter of the filtering medium for selective adsorption of blood components is 0.1 to 50 $\mu$m, and is preferably 4.5 to 30 $\mu$m and more preferably 5 to 15 $\mu$m from the reason of improving a selective removal rate of blood components (particularly a leukocyte).

Herein, the "average through-hole diameter" is evaluated by increasing air pressure to 2 cc/min with respect to a sample completely wetted by GALWTCK (manufactured by Porous Materials, Inc.) in a pore size distribution measurement test using a perm porometer (CFE-1200 AEX manufactured by Seika Corporation), in the same manner as in the method described in paragraph <0093> of JP2012-046843A. Specifically, with respect to a membrane sample completely wetted by GALWICK, a certain amount of air was fed at 2 cc/min to one side of the membrane, and while measuring a pressure thereof, a flow rate of air permeating to an opposite side of the membrane is measured. In this method, firstly, data on pressure and permeated air flow rate (hereinafter referred to as a "wet curve") is obtained for the membrane sample wetted by GALWICK. Next, the same data (hereinafter referred to as a "dry curve") was measured for the membrane sample in a dry state, and a pressure at an intersection of a curve (half dry curve) corresponding to half of a dry curve flow rate and the wet curve is calculated. Thereafter, an average through-hole diameter can be calculated by introducing a surface tension ($\gamma$) of GALWICK, a contact angle ($\theta$) with the filtering medium, and an air pressure (P) into Formula (I).

$$\text{Average through-hole diameter} = 4\gamma\cos\theta/\text{P} \qquad \text{(I)}$$

In addition, a method for adjusting the "average through-hole diameter" is not particularly limited. For example, in a case where a form of the filtering medium for adsorption of the embodiment of the present invention is a nonwoven fabric made of nanofibers, in a case where a plurality of nozzles are used upon spinning using a nanofiber manufacturing apparatus, a part of the nozzles is used as a nozzle for supplying a polyvinyl alcohol aqueous solution, and a hole diameter can be adjusted by adjusting the number of these nozzles. That is, after the nonwoven fabric made of cellulose acylate and polyvinyl alcohol is produced, the hole diameter can be adjusted by immersing the nonwoven fabric in water to dissolve the polyvinyl alcohol.

In the present invention, a specific surface area of the filtering medium for selective adsorption of blood components is 1.0 to 100 m$^2$/g, but is preferably 1 to 50 m$^2$/g and more preferably 3 to 50 m$^2$/g from the reason of further improving the selective removal rate of blood components (particularly a leukocyte).

Herein, the "specific surface area" refers to a measurement value measured by Brunauer-Emmett-Teller (BET) method by nitrogen adsorption.

In addition, the "specific surface area" can be appropriately adjusted by, for example, a fiber diameter of a fiber made of cellulose acylate, impartment of unevenness to a fiber surface, and the like.

In the present invention, a thickness of the filtering medium for selective adsorption of blood components is not particularly limited, but may be 5 $\mu$m to 30,000 $\mu$m, preferably 10 $\mu$m to 3,000 $\mu$m, and more preferably 10 $\mu$m to 500 $\mu$m from the viewpoint of imparting a sufficient space volume and a sufficient mechanical strength which are for allowing the filtering medium to hold a subject to be removed, from the viewpoint of compatibility between a low filtration pressure and a high separation capacity, and the like.

The thickness can be obtained by measuring a membrane thickness of the filtering medium at 10 points using a micrometer (manufactured by Mitutoyo) and averaging the respective measured values.

[Cellulose acylate]

In the present invention, a substitution degree with an acyl group included in the cellulose acylate is preferably within a range of 1.00 to 2.90, more preferably within a range of 2.00 to 2.90, and even more preferably within a range of 2.80 to 2.90 from the reason of further improving a selective removal rate of blood components (particularly a leukocyte).

Herein, the "substitution degree" refers to a substitution degree of the hydrogen atoms that constitute hydroxyl groups of cellulose with acyl groups (hereinafter will also be referred to as "acylation degree"), and the substitution degree can be calculated by comparing the area intensity ratio of carbons of cellulose acylate measured by a $^{13}$C-NMR method.

In addition, the "substitution degree" of the acyl group can be appropriately adjusted by a method in which a partial hydrolysis time is changed when synthesizing the cellulose acylate, a method in which alkaline saponification is performed after producing a nonwoven fabric or a porous membrane, and the like.

Specific examples of the acyl group include an acetyl group, a propionyl group, a butyryl group, and the like.

The acyl groups to be substituted may be composed only of a single kind (for example, only an acetyl group) or may be of two or more kinds.

In the present invention, from the reason of improving uniformity of a fiber diameter and improving the appearance in a case where a nonwoven fabric is produced, the number of carbon atoms of the acyl group included in the cellulose acylate is preferably 4 or less, and a acetyl group is preferable.

The cellulose acylate in which the acyl group is the acetyl group will also be referred to as "cellulose acetate" in the following explanation.

A number average molecular weight (Mn) of the cellulose acylate is not particularly limited, but is preferably 40,000 or more, more preferably 40,000 to 150,000, and even more preferably 60,000 to 100,000 from the viewpoint of strength.

In addition, a weight-average molecular weight (Mw) of the cellulose acylate is not particularly limited, but is preferably 100,000 or more, more preferably 100,000 to 500,000, and even more preferably 150,000 to 300,000 from the viewpoint of the mechanical strength of the fiber composite.

The weight-average molecular weight or number average molecular weight according to the present specification is a value measured by a gel permeation chromatography (GPC) method under the following conditions.

- Apparatus name: HLC-8220 GPC (Tosoh Corporation)
- Type of column: TSK gel Super HZ4000 and HZ2000 (Tosoh Corporation)
- Eluent: Dimethylformamide (DMF)
- Flow rate: 1 ml/min
- Detector: RI
- Sample concentration: 0.5%
- Calibration curve base resin: TSK standard polystyrene (molecular weights 1,050, 5,970, 18,100, 37,900, 190,000, and 706,000)

<Method for synthesizing cellulose acylate>

Regarding a method for synthesizing the cellulose acylate as described above, the description in p. 7 to 12 of Hatsumei Suishin Kyokai Kokai Giho (Journal of Technical Disclosure) (Technology No. 2001-1745, published on March 15, 2001, Japan Institute for Promoting Invention and Innovation) is also applicable.

(Raw material)

Regarding a raw material of cellulose, suitable examples include raw materials originating from hardwood pulp, softwood pulp, cotton linter, and the like. Among them, raw materials originating from cotton linter are preferred because an amount of hemicellulose is small, and a nanofiber having further improved uniformity of the fiber diameter can be produced.

(Activation)

It is preferable that the raw material of cellulose is subjected to a treatment of contacting with an activating agent (activation), prior to acylation.

Specific examples of the activating agent include acetate, propionic acid, butyric acid, and the like, and among them, acetate is preferred.

The amount of addition of the activating agent is preferably 5% to 10,000%, more preferably 10% to 2,000%, and even more preferably 30% to 1,000%.

The method for addition can be selected from methods such as spraying, dropwise addition, and immersion.

Activation time is preferably 20 minutes to 72 hours, and more preferably 20 minutes to 12 hours.

The activation temperature is preferably 0°C to 90°C, and more preferably 20°C to 60°C.

Furthermore, an acylation catalyst such as sulfuric acid may be added to the activating agent, in an amount of 0.1% to 10% by mass.

(Acylation)

It is preferable for synthesizing a uniform cellulose acylate that the hydroxyl groups of cellulose are acylated by reacting cellulose with an acid anhydride of a carboxylic acid using Brønsted acid or a Lewis acid (see "Rikagaku Shoten (Dictionary of Physics and Chemistry," 5th Edition (2000)) as a catalyst, and control of the molecular weight is also enabled.

Examples of the method for obtaining a cellulose acylate include a method of causing a reaction by adding two kinds of carboxylic acid anhydrides as acylating agents as a mixture or in sequence to the system; a method of using a mixed acid anhydride of two kinds of carboxylic acids (for example, a mixed acid anhydride of acetate and propionic acid); a method of forming a mixed acid anhydride (for example, an acid anhydride of acetate and propionic acid) within the reaction system by using acid anhydrides of a carboxylic acid and another carboxylic acid (for example, acid anhydrides of acetate and propionic acid) as raw materials, and reacting the mixed acid anhydride with cellulose; and a method of first synthesizing a cellulose acylate having a substitution degree of less than 3, and further acylating residual hydroxyl groups by using an acid anhydride or an acid halide.

Furthermore, in regard to the synthesis of a cellulose acylate having a high degree of the 6-position substitution, the details are described in JP1999-005851A (JP-H11-005851A), JP2002-212338A, JP2002-338601A, and the like.

<Acid anhydride>

The acid anhydride of a carboxylic acid is preferably an acid anhydride of a carboxylic acid having 2 to 6 carbon atoms, and specifically, suitable examples include acetic anhydride, propionic anhydride, butyric anhydride, and the like.

It is preferable that the acid anhydride is added in an amount of 1.1 to 50 equivalents, more preferably 1.2 to 30 equivalents, and even more preferably 1.5 to 10 equivalents, with respect to the hydroxyl groups of cellulose.

<Catalyst>

Regarding the acylation catalyst, it is preferable to use a Brønsted acid or a Lewis acid, and it is more preferable to use sulfuric acid or perchloric acid.

The amount of addition of the acylation catalyst is preferably 0.1% to 30% by mass, more preferably 1% to 15% by mass, and even more preferably 3% to 12% by mass.

<Solvent>

Regarding the acylation solvent, it is preferable to use a carboxylic acid, and it is more preferable to use a carboxylic acid having from 2 to 7 carbon atoms. Specifically, it is even more preferable to use, for example, acetate, propionic acid, butyric acid, or the like. These solvents may also be used as mixtures.

<Condition>

In order to control a temperature increase caused by the heat of reaction of acylation, it is preferable that the acylating agent is cooled in advance.

An acylation temperature is preferably -50°C to 50°C, more preferably -30°C to 40°C, and even more preferably -20°C to 35°C.

A minimum temperature of the reaction is preferably -50°C or higher, more preferably -30°C or higher, and even more preferably -20°C or higher.

An acylation time is preferably 0.5 hours to 24 hours, more preferably 1 hour to 12 hours, and even more preferably 1.5 hours to 10 hours.

Adjustment of the molecular weight is enabled by controlling the acylation time.

<Reaction terminating agent>

It is preferable that a reaction terminating agent is added after the acylation reaction.

The reaction terminating agent may be any compound capable of decomposing an acid anhydride, and specific examples thereof include water, an alcohol having 1 to 3 carbon atoms, and a carboxylic acid (for example, acetate, propionic acid, butyric acid, or the like). Above all, a mixture of water and a carboxylic acid (acetate) is preferred.

A composition of water and the carboxylic acid is such that a content of water is preferably 5% to 80% by mass, more preferably 10% to 60% by mass, and even more preferably 15% to 50% by mass.

<Neutralizing agent>

After termination of the acylation reaction, a neutralizing agent may be added.

Examples of the neutralizing agent include ammonium, organic quaternary ammoniums, alkali metals, metals of Group 2, metals of Groups 3 to 12, and carbonates, hydrogen carbonates, organic acid salts, hydroxides, or oxides of the elements of Groups 13 to 15. Specifically, suitable examples include carbonate, hydrogen carbonate, acetate, or hydroxide of sodium, potassium, magnesium, or calcium.

<Partial hydrolysis>

The cellulose acylate obtained by the acylation described above has a total substitution degree of almost 3; however, for the purpose of adjusting the substitution degree to a desired value (for example, degree of about 2.8), the acyl substitution degree of the cellulose acylate can be decreased to a desired extent, by partially hydrolyzing ester bonds by maintaining the cellulose acylate for several minutes to several days at 20°C to 90°C in the presence of water and a small amount of catalyst (for example, an acylation catalyst such as residual sulfuric acid). Meanwhile, partial hydrolysis can be terminated as appropriate using residual catalyst and the neutralizing agent.

<Filtration>

Filtration may be carried out in any process between the completion of acylation and reprecipitation. It is also preferable to dilute the system with an appropriate solvent prior to filtration.

<Reprecipitation>

A cellulose acylate solution can be mixed with water or an aqueous solution of a carboxylic acid (for example, acetate, propionic acid, or the like), and thus reprecipitation can be induced. Reprecipitation may be any of continuous type or batch type.

<Washing>

After reprecipitation, it is preferable to perform a washing treatment. Washing is carried out using water or warm water, and completion of washing can be checked through the pH, ion concentration, electrical conductivity, elemental analysis, or the like.

<Stabilization>

It is preferable that a weak alkali (carbonate, hydrogen carbonate, hydroxide, or oxide of Na, K, Ca, Mg, or the like) is added to the cellulose acylate obtained after washing, for the purpose of stabilization.

<Drying>

It is preferable that the cellulose acylate is dried at 50°C to 160°C until a percentage moisture content reaches 2% by mass or less.

In the present invention, it is preferable that the above-mentioned cellulose acylate constitutes at least a part of the surface of the member that comes into contact with blood, for the reason of further improving the selective removal rate of blood components (particularly a leukocyte). It is considered that this is because an adsorption performance of the cellulose acylate itself to a blood component is easily exhibited.

Herein, the "surface of the member that comes into contact with blood" refers to a surface of a portion where the filtering medium for adsorption of the embodiment of the present invention comes into contact with blood. For example, in a case where the filtering medium for adsorption of the embodiment of the present invention is a nonwoven fabric, the surface refers to a surface of a fiber constituting the nonwoven fabric; and in a case where the filtering medium for adsorption of the embodiment of the present invention is a porous membrane, the surface refers to a surface of the porous membrane and a wall surface of an internal cavity.

The filtering medium for adsorption of the embodiment of the present invention is preferably in the form of a nonwoven fabric or a porous membrane, and is more preferably a nonwoven fabric.

In addition, as a nonwoven fabric, a nonwoven fabric made of fibers having an average fiber diameter of 1 nm to 5 $\mu$m and an average fiber length of 1 mm to 1 m is preferable, a nonwoven fabric made of nanofibers having an average fiber diameter of 100 nm and or more and less than 1,000 nm and an average fiber length of 1.5 mm to 1 m is more preferable, and a nonwoven fabric made of nanofibers having an average fiber diameter of 100 nm to 800 nm and an average fiber length of 2.0 mm to 1 m is even more preferable.

The average fiber diameter and average fiber length can be adjusted by adjusting a concentration of a cellulose acylate solution in a case of producing the nonwoven fabric.

The average fiber diameter means a value measured as follows.

The surface of a nonwoven fabric formed from fibers is observed by taking a Transmission Electron Microscope (TEM) image or a Scanning Electron Microscope (SEM) image.

An observation based on an electron microscopic image is performed at a magnification ratio selected from 1,000 times to 5,000 times depending on the size of the constituent fiber. However, the sample, the observation conditions, and the magnification ratio are adjusted so as to satisfy the following conditions.

(1) One straight line X is drawn at any site within an image to be observed, and 20 or more fibers intersect this straight line X.
(2) A straight line Y perpendicularly intersecting the straight line X is drawn in the same image, and 20 or more fibers intersect the straight line Y

In regard to the electron microscopic observation images such as described above, for each of the fibers intersecting the straight line X and the fibers intersecting the straight line Y, widths (minor axis of the fiber) of at least 20 fibers (that is, at least 40 fibers in total) are read out. In this manner, an observation of at least 3 sets or more of the electron microscopic images such as described above is made, and fiber diameters of at least 40 fibers $\times$ 3 sets (that is, at least 120 fibers) are read out.

The average fiber diameter is determined by averaging the fiber diameters read out as such.

The average fiber length means a value measured as follows.

That is, the fiber length of the fiber can be determined by analyzing the electron microscopic observation image used on the occasion of measuring the average fiber diameter described above.

Specifically, in the electron microscopic observation image such as described above, for each of the fibers intersecting the straight line X and the fibers intersecting the straight line Y, fiber lengths of at least 20 fibers (that is, at least 40 fibers in total) are read out.

In this manner, an observation of at least 3 sets or more of the electron microscopic images such as described above is made, and the fiber lengths of at least 40 fibers $\times$ 3 sets (that is, at least 120 fibers) are read out.

The average fiber length is determined by averaging the fiber lengths read out as such.

<Manufacture process of filtering medium>

In the present invention, a method for producing the nanofiber is not particularly limited; however, a production method utilizing an electrospinning method (hereinafter will also be referred to as "electrospinning method") is preferable, and the nanofiber can be produced by, for example, discharging a solution obtained by dissolving the above-described cellulose acylate in a solvent, from a distal end of a nozzle at a constant temperature in the range of from 5°C to 40°C, applying a voltage between the solution and a collector, and jetting out fibers from the solution into the collector. Specifically, the nanofiber can be produced by a method shown in paragraphs <0014> to <0044> and Figs. 1 and 2 of JP2016-053232A, and the like.

In addition, a method for producing the nonwoven fabric is not limited, and a nonwoven fabric 120 can be manufactured by a nanofiber manufacturing apparatus 110 shown in Fig. 1 of JP2016-053232A, for example.

Meanwhile, a method for producing a porous membrane is not limited. For example, using a coating solution containing a cellulose acylate, a good solvent in which the cellulose acylate dissolves, a solvent in which the cellulose acylate does not dissolve but the cellulose acylate dissolves, and a poor solvent which easily causes phase separation, the poor solvent is phase-separated at the same time when the dissolved cellulose acylate precipitates at the time of drying after coating, the poor solvent is evaporated to be removed, and therefore a porous membrane having holes formed therein can be formed.

<Fiber adhesion treatment process of filtering medium>

In a process of manufacturing the filtering medium for selective adsorption of blood components of the embodiment of the present invention, in order to make fibers adhere to each other, a heat fusion welding process or an adhesion treatment process may be included during a process of manufacturing the nonwoven fabric or after a process of manufacturing the nonwoven fabric by using a nanofiber manufacturing apparatus.

The filtering medium for adsorption of the embodiment of the present invention is preferably used as a filtering medium which selectively adsorbs a blood cell component, and is more preferably used as a filtering medium which selectively adsorbs a leukocyte component, among blood components.

[Blood filter]

A blood filter of the embodiment of the present invention is a blood filter filled with the above-described filtering medium for selective adsorption of blood components of the embodiment of the present invention.

Examples of such a blood filter include, in a case where the filtering medium for selective adsorption of blood components is in the form of a nonwoven fabric, a module in which a nonwoven fabric is set in a housing in a flat membrane shape, a spiral-type module in which a filtering medium formed into a cylindrical nonwoven fabric is set in a housing.

Examples

Hereinafter, the present invention will be described in more detail based on Examples. The materials, amounts used, proportions, treatments, treatment procedures, and the like disclosed in the following Examples can be modified as appropriate as long as the gist of the invention is maintained. Therefore, the scope of the invention should not be limitedly interpreted by the Examples described below.

[Examples 1 to 3]

<Synthesis of cellulose acetate>

Cellulose (raw material: cotton linter) was mixed with acetate and sulfuric acid, and the mixture was acetylated while a reaction temperature was maintained at 40°C or lower.

After the raw material cellulose disappeared and acetylation was completed, the system was further heated continuously at a temperature of 40°C or lower, and the degree of polymerization was adjusted to a desired value.

Next, residual acid anhydride was hydrolyzed by adding an aqueous solution of acetate, and then partial hydrolysis was performed by heating at a temperature of 60°C or lower. Thus, the substitution degree was adjusted as shown in Table 1.

Residual sulfuric acid was neutralized with an excess amount of magnesium acetate. Reprecipitation from the aqueous solution of acetate was performed, and washing with water was repeated. Thus, a cellulose acetate was synthesized.

<Production of nonwoven fabric>

The cellulose acetate thus synthesized was dissolved in a mixed solvent of 88% of dichloromethane and 12% of methanol to prepare a cellulose acetate solution having a concentration of 3.5 g/100 cm$^3$. Therefore, a nonwoven fabric having a size of 20 $\times$ 30 cm and formed from cellulose acetate nanofibers was produced using a nanofiber manufacturing apparatus having a plurality of nozzles, and used as a filtering medium for selective adsorption of blood components.

[Example 4 and Comparative Example 1]

A nonwoven fabric formed from cellulose acetate nanofibers was produced in the same manner as in Example 1 to be used as a filtering medium for selective adsorption of blood components, except that, in a part of nozzles in a nanofiber manufacturing apparatus, spinning is performed using a polyvinyl alcohol (PVA) aqueous solution instead of the cellulose acetate solution, and the part of nozzles is immersed in water after spinning to remove PVA in the case of producing a nonwoven fabric.

[Examples 5 and 6 and Comparative Example 2]

A nonwoven fabric formed from cellulose acetate nanofibers was produced in the same manner as in Example 1 to be used as a filtering medium for selective adsorption of blood components, except that a substitution degree was adjusted as shown in Table 1.

[Example 7]

A nonwoven fabric formed from cellulose propionate nanofibers was produced in the same manner as in Example 1 to be used as a filtering medium for selective adsorption of blood components, except that an acyl group was changed from an acetyl group to a propionyl group, and a substitution degree was adjusted as shown in Table 1.

[Example 8]

A nonwoven fabric formed from cellulose butanoate nanofibers was produced in the same manner as in Example 1 to be used as a filtering medium for selective adsorption of blood components, except that an acyl group was changed from an acetyl group to a butyryl group, and a substitution degree was adjusted as shown in Table 1.

[Comparative Example 3]

A nonwoven fabric formed from carboxymethylcellulose nanofibers was produced in the same manner as in Example 1 to be used as a filtering medium for selective adsorption of blood components, except that carboxymethylcellulose having a substitution degree shown in Table 1 was used instead of cellulose acetate.

[Comparative Example 4]

A nonwoven fabric formed from ethyl cellulose nanofibers was produced in the same manner as in Example 1 to be used as a filtering medium for selective adsorption of blood components, except that ethyl cellulose having a substitution degree shown in Table 1 was used instead of cellulose acetate, and methyl ethyl ketone was used instead of a mixed solvent.

[Examples 9 to 11]

A nonwoven fabric formed from cellulose acetate nanofibers was produced in the same manner as in Example 1 to be used as a filtering medium for selective adsorption of blood components, except that a substitution degree was adjusted as shown in Table 1.

[Comparative Example 5]

After producing the nanofiber nonwoven fabric in the same manner as in Example 1, the nanofiber nonwoven fabric was immersed in an aqueous solution of 0.5 N sodium hydroxide to which 5% ethanol was added, for 48 hours.
After immersion, the nanofiber nonwoven fabric was immersed in pure water, then washed and dried, and therefore a nonwoven fabric made from deacylated cellulose was produced and used as a filtering medium for selective adsorption of blood components. Because a substitution degree of cellulose acetate deacylated under the same conditions is 0.00, a substitution degree in Comparative Example 5 in Table 1 is denoted as "0.00" because of non-substitution.

[Example 12]

<Preparation of dope>

A dope having a composition shown below was prepared.
Specifically, cellulose acetate was dissolved in dimethyl chloride, and methanol was added to this solution little by little. Next, glycerin and pure water were added little by little to this solution to prepare a solution in a state of having almost no undissolved matter. The solution was filtered through a filter paper, and therefore a dope was prepared.

(Dope composition)

| · Cellulose acetate (acetylation degree: 60.8%) | 5 parts by mass |
| · Glycerin | 0.2 parts by mass |
| · Dimethylchloride | 55 parts by mass |
| · Methanol | 34 parts by mass |
| · Pure water | 6 parts by mass |

<Production of porous membrane>

The prepared dope was sent by a gear pump, further filtrated, and then cast from a die onto a polyethylene terephthalate (PET) film which was carried on an endless band and conveyed.
This cast membrane was dried for 20 minutes with drying air at 20°C to 40°C.
From the endless band, the film was peeled off with PET. The peeled off film was dried for 15 minutes at 80°C to 120°C with hot air and wound with a winder. In the cellulose acetate on PET, a large number of fine pores were formed.
Using a peeling bar, the cellulose acetate microporous membrane was separated from PET and used as a filtering medium for selective adsorption of blood components.

[Comparative Example 6]

A nonwoven fabric formed from cellulose acetate nanofibers was produced in the same manner as in Example 1 to be used as a filtering medium for selective adsorption of blood components, except that, in a part of nozzles in a nanofiber manufacturing apparatus, spinning is performed using a polyvinyl alcohol (PVA) aqueous solution instead of the cellulose acetate solution, and the part of nozzles is immersed in water after spinning to remove PVA in the case of producing a nonwoven fabric.

[Comparative Example 7]

A filtering medium for selective adsorption of blood components was obtained in the same manner as in Example 1 except that a thickness of the nonwoven fabric was increased 5 times and the produced nonwoven fabric was subjected to a compress process at a pressure of 1 MPa in a membrane thickness direction.

[Comparative Example 8]

Polyacrylonitrile was dissolved in N,N-dimethylformamide (DMF) to prepare 3.5 g/100 cm$^3$ polyacrylonitrile. Therefore, a nonwoven fabric having a size of 20 × 30 cm and formed from polyacrylonitrile nanofibers was produced using a nanofiber manufacturing apparatus having a plurality of nozzles, and used as a filtering medium for selective adsorption of blood components.

[Comparative Example 9]

A nonwoven fabric formed from polyacrylonitril nanofibers was produced in the same manner as in Example 8 to be used as a filtering medium for selective adsorption of blood components, except that, in a part of nozzles in a nanofiber manufacturing apparatus, spinning is performed using a polyvinyl alcohol (PVA) aqueous solution instead of the cellulose acetate solution, and the part of nozzles is immersed in water after spinning to remove PVA in the case of producing a nonwoven fabric.

[Comparative Example 10]

Dissolution was performed in NMP so that polyurethane became 14% by mass, methylcellulose became 14% by mass, and calcium chloride dihydrate became 1.4% by mass.
Next, this solution was coated on a PET film and immersed in a 50% by mass NMP aqueous solution at 50°C to coagulate.
Next, the film was washed with pure water and dried in an oven at 55°C to produce a polyurethane porous membrane, which was used as a filtering medium for selective adsorption of blood components.

[Comparative Example 11]

Polypropylene was dissolved in a mixed solvent having a mass ratio of decalin, acetone, and DMF of 8:1:1 so as to become 12.5% by mass. Therefore, a nonwoven fabric having a size of 20 × 30 cm and formed from polypropylene was produced using a nanofiber manufacturing apparatus having a plurality of nozzles, and used as a filtering medium for selective adsorption of blood components.

[Comparative Example 12]

With reference to a method disclosed in JP1993-087808A (JP-H05-087808A), cellulose acetate pellets were injection molded to form beads of 3.2 mmφ, followed by five extraction operations at 50°C for 1 hour in methanol. The pellets were air-dried for 15 hours and further dried at 80°C for 5 hours to obtain a filtering medium for selective adsorption of blood components.

[Example 13]

Cellulose acetate propionate (CAP-482-20 manufactured by Eastman Chemical Company) was dissolved in a mixed solvent of 88% dichloromethane and 12% methanol to prepare a cellulose acetate propionate solution of 7.5 g/100 cm$^3$. Using a nanofiber manufacturing apparatus having a plurality of nozzles, a nonwoven fabric made of cellulose acetate propionate fibers of 20 × 30 cm was produced.
Next, the prepared nonwoven fabric was treated at 200°C for 5 minutes using a constant temperature drier, and then taken out to produce a filtering medium for selective adsorption of blood components.

[Example 14]

Cellulose acetate butyrate (CAB-381-20 manufactured by Eastman Chemical Company) was dissolved in a mixed solvent of 88% dichloromethane and 12% methanol to prepare a cellulose acetate butyrate solution of 7.5 g/100 cm$^3$. Using a nanofiber manufacturing apparatus having a plurality of nozzles, a nonwoven fabric made of cellulose acetate butyrate fibers of 20 × 30 cm was produced.
Next, the prepared nonwoven fabric was treated at 180°C for 5 minutes using a constant temperature drier, and then taken out to produce a filtering medium for selective adsorption of blood components.

[Example 15]

A nonwoven fabric formed of cellulose acetate fiber and cellulose acetate propionate fiber was produced a solution to be supplied to a part of the nozzles in the nanofiber manufacturing apparatus of Example 13 was used instead of the cellulose acetate propionate solution, and the cellulose acetate of Example 1 was dissolved in a mixed solvent of 88% dichloromethane and 12% methanol to obtain a solution of 5.8 g/100 cm$^3$ to be subjected to spinning. The number ratio of the nozzles supplied with each solution was set to 2:1 of cellulose acetate solution: cellulose acetate propionate solution.
Next, the prepared nonwoven fabric was treated at 230°C for 5 minutes using a constant temperature drier, and then taken out to produce a filtering medium for selective adsorption of blood components.
A material of the produced filtering medium for selective adsorption of blood components, a thickness of the filtering medium, a substitution degree, a form, an average fiber diameter, an average fiber length, a glass transition temperature, an average through-hole diameter, and a specific surface area are shown in Table 1. A substitution degrees of CAP and CAB described in Examples 13 to 15 are shown in Table 2.
In Table 1, materials shown in alphabetical notation are the materials as shown below.

CA: cellulose acetate
CP: Cellulose propionate
CB: cellulose butyrate
CMC: Carboxymethylcellulose
EC: Ethyl cellulose
PU: Polyurethane
PAN: Polyacrylonitrile
PP: Polypropylene
CAP: Cellulose acetate propionate
CAB: Cellulose acetate butyrate

[Evaluation]

Before carrying out evaluations described below, high-pressure steam sterilization treatment was performed on the produced filtering medium for selective adsorption of blood components under conditions of 134°C for 30 minutes, using a high pressure steam sterilizer.

<Removal rate of leukocytes>

A syringe was set up so that a flange portion faces upward, and the filtering medium was set at the bottom part.
Next, 1.5 ml of human whole blood was added dropwise, filtered under a constant pressure, collected from a lower part of the syringe. The number of leukocytes before and after passing through the filtering medium was obtained by blood analysis, and a removal rate of leukocytes was calculated from the following equation. The results are shown in Table 1.
Removal rate of leukocytes = [1- (number of leukocytes after passing through filtering medium/number of leukocytes before passing through filtering medium)] $\times$ 100%

<Clogging>

A syringe was set up so that a flange portion faces upward, and the filtering medium was set at the bottom part.
Next, 1.5 ml of human whole blood was added dropwise, a constant pressure was applied, and filtered. 20 ml of the blood was further filtered.
Next, a filtration rate in a case where 1.5 ml was filtered again was measured and compared with the initial 1.5 ml filtration rate to carry out evaluation in the following three stages. The results are shown in Table 1.

1: 1 time or more and less than 1.2 times the initial rate
2: 1.2 times or more and less than 2 times the initial rate
3: 2 or more times the initial rate

[Table 1]

| | Material | Substitution degree | Form | Thickness | Average fiber diameter | Average fiber length | Glass transition temperature | Average through-hole diameter | Specific surface area | Removal rate of leukocytes | Clogging |
|---|---|---|---|---|---|---|---|---|---|---|---|
| | | | | $\mu$m | nm | mm | °C | $\mu$m | m²/g | % | - |
| Example 1 | CA | 2.88 | Nonwoven fabric | 67 | 516 | 4.5 | 177 | 9.9 | 6.9 | 100 | 1 |
| Example 2 | CA | 2.88 | Nonwoven fabric | 68 | 520 | 4.5 | 177 | 5.7 | 7.3 | 100 | 1 |
| Example 3 | CA | 2.88 | Nonwoven fabric | 59 | 515 | 4.4 | 177 | 27.3 | 6.0 | 96 | 1 |
| Example 4 | CA | 2.88 | Nonwoven fabric | 65 | 503 | 4.4 | 177 | 4.4 | 7.0 | 100 | 2 |
| Comparative Example 1 | CA | 2.88 | Nonwoven fabric | 60 | 525 | 4.4 | 177 | 51.3 | 5.5 | 86 | 1 |
| Example 5 | CA | 2.43 | Nonwoven fabric | 86 | 114 | 3.6 | 192 | 106 | 44.3 | 100 | 1 |
| Example 6 | CA | 2.43 | Nonwoven fabric | 72 | 987 | 2.2 | 192 | 106 | 1.3 | 96 | 1 |
| Comparative Example 2 | CA | 2.43 | Nonwoven fabric | 77 | 994 | 2.3 | 192 | 10.8 | 0.9 | 91 | 1 |
| Example 7 | CP | 2.33 | Nonwoven fabric | 69 | 635 | 3.9 | 154 | 6.6 | 5.6 | 98 | 1 |
| Example 8 | CB | 2.02 | Nonwoven fabric | 70 | 622 | 4.0 | 138 | 7.8 | 5.7 | 96 | 1 |
| Comparative Example 3 | CMC | 1.41 | Nonwoven fabric | 66 | 531 | 3.3 | *1 | 8.0 | 5.8 | 90 | 1 |
| Comparative Example 4 | EC | 1.30 | Nonwoven fabric | 61 | 627 | 3.8 | 134 | 9.8 | 5.0 | 89 | 1 |
| Example 9 | CA | 2.01 | Nonwoven fabric | 69 | 488 | 4.3 | 201 | 10.1 | 6.5 | 98 | 1 |

EP 3 549 623 B1

14

(continued)

| | Material | Substitution degree | Form | Thickness | Average fiber diameter | Average fiber length | Glass transition temperature | Average through-hole diameter | Specific surface area | Removal rate of leukocytes | Clogging |
|---|---|---|---|---|---|---|---|---|---|---|---|
| | | | | μm | nm | mm | °C | μm | m²/g | % | - |
| Example 10 | CA | 1.12 | Nonwoven fabric | 67 | 479 | 4.2 | 215 | 100 | 6.6 | 96 | 1 |
| Example 11 | CA | 0.50 | Nonwoven fabric | 67 | 473 | 4.2 | 225 | 10.2 | 6.7 | 95 | 1 |
| Comparative Example 5 | Cellulose | 0.00 | Nonwoven fabric | 68 | 466 | 4.1 | 240 | 10.3 | 6.8 | 88 | 1 |
| Example 12 | CA | 2.88 | Porous membrane | 231 | - | - | 177 | 8.9 | 6.3 | 100 | 1 |
| Comparative Example 6 | CA | 2.88 | Nonwoven fabric | 58 | 514 | 4.1 | 177 | 108.9 | 5.7 | 36 | 1 |
| Comparative Example 7 | CA | 2.88 | Nonwoven fabric | 79 | 521 | 4.1 | 177 | 009 | 6.1 | 100 | 3 |
| Comparative Example 8 | PAN | - | Nonwoven fabric | 33 | 557 | 3.8 | 103 | 5.5 | 5.2 | 100 | 3 |
| Comparative Example 9 | PAN | - | Nonwoven fabric | 30 | 567 | 3.8 | 103 | 9.5 | 5.0 | 67 | 2 |
| Comparative Example 10 | PU | - | Porous membrane | 289 | - | - | -51 | 7.5 | 1.8 | *2 | *2 |
| Comparative Example 11 | PP | - | Nonwoven fabric | 367 | 831 | 2.1 | 0 | 3.4 | 3.3 | 100 | 3 |
| Comparative Example 12 | CA | 2.27 | 3.2 mmφ beads | - | - | - | 195 | - | 0.05 | 54 | 1 |
| Example 13 | CAP | *3 | Nonwoven fabric | 175 | 1529 | 5.8 | 149 | 7.1 | 4.3 | 98 | 1 |
| Example 14 | CAB | *3 | Nonwoven fabric | 181 | 1261 | 6.3 | 142 | 8.6 | 5.1 | 98 | 1 |

| | Material | Substitution degree | Form | Thickness | Average fiber diameter | Average fiber length | Glass transition temperature | Average through-hole diameter | Specific surface area | Removal rate of leukocytes | Clogging |
|---|---|---|---|---|---|---|---|---|---|---|---|
| | | | | μm | nm | mm | °C | μm | m²/g | % | - |
| Example 15 | CA/CAP | CA(2.88) /CAP (*3) | Nonwoven fabric | 222 | 1756 | 5.9 | 177/149*4 | 9.0 | 4.4 | 97 | 1 |

* 1: A glass transition temperature could not be checked.

*2: Blood did not permeate the filtering medium, and thus could not be filtered.

*3: Substitution degrees of CAP or CAB described in Table 2 were used.

*4: Each of glass transition temperatures of CA and CAP was checked.

[Table 2]

| | | Substitution degree | | Remarks |
|---|---|---|---|---|
| | | Acetyl group | Propionyl group | Butanoyl group | |

| | Acetyl group | Propionyl group | Butanoyl group | Remarks |
|---|---|---|---|---|
| | **Substitution degree** | | | |
| CAP | 0.15 | 2.63 | 0 | CAP-482-20 manufactured by Eastman Chemical Company |
| CAB | 1.00 | 0 | 1.70 | CAB-381-20 manufactured by Eastman Chemical Company |

Based on the results shown in Table 1, it was found that, in a case where a nonwoven fabric having an average through-hole diameter of more than 50 $\mu$m was used as a filtering medium for selective adsorption of blood components, a removal rate of leukocytes was poor (Comparative Example 1).

In addition, it was found that, in a case where a nonwoven fabric having a specific surface area of less than 1.0 m$^2$/g was used as a filtering medium for selective adsorption of blood components, a removal rate of leukocytes was poor (Comparative Example 2).

Furthermore, it was found that, in a case where a nonwoven fabric containing a cellulosic material not corresponding to cellulose acylate was used as a filtering medium for selective adsorption of blood components, a removal rate of leukocytes was poor (Comparative Examples 3 to 5).

Furthermore, it was found that, in a case where a nonwoven fabric having an average through-hole diameter outside the range of 0.1 to 50 $\mu$m is used as a filtering medium for selective adsorption of blood components, a removal rate of leukocytes was poor, or clogging occurred (Comparative Example 6 and 7).

Furthermore, it was found that, in a case where a nonwoven fabric containing materials other than cellulosic materials was used as a filtering medium for selective adsorption of blood components, a removal rate of leukocytes was poor, or clogging occurred (Comparative Examples 8 to 11). This is considered to be mainly caused by deformation of the filtering medium due to the high-pressure steam sterilization treatment.

Furthermore, it was found that the filtering medium formed of beads having no through-holes and having a specific surface area of less than 1.0 m$^2$/g was poor in a removal rate of leukocytes (Comparative Example 12).

With respect to the above results, it was found that, in a case where a porous membrane or a nonwoven fabric containing cellulose acylate, and having a glass transition temperature of 126°C or higher, an average through-hole diameter of 0.1 to 50 $\mu$m, and a specific surface area of 1.0 to 100 m$^2$/g was used as a filtering medium for selective adsorption of blood components, a removal rate of leukocytes increased while suppressing clogging (Examples 1 to 15).

## Claims

1. Non-therapeutic use of a filtering medium for selective adsorption of blood components, the filtering medium containing a cellulose acylate, wherein

   a glass transition temperature of the filtering medium is 126°C or higher,
   an average through-hole diameter is 0.1 to 50 $\mu$m, and
   a specific surface area is 1.0 to 100 m$^2$/g, wherein
   the glass transition temperature and the average through-hole diameter are determined as defined in the description; and
   the specific surface area is measured by the Brunauer-Emmett-Teller method by nitrogen adsorption.

2. Use according to claim 1, wherein a substitution degree of an acyl group included in the cellulose acylate is within a range of 1.00 to 2.90, wherein the substitution degree is calculated by comparing the area intensity ratio of carbons of cellulose acylate measured by a $^{13}$C-NMR method

3. Use according to claim 1 or 2, wherein the acyl group included in the cellulose acylate has 4 or less carbon atoms.

4. Use according to any one of claims 1 to 3, wherein the acyl group included in the cellulose acylate includes at least an acetyl group.

5. Use according to any one of claims 1 to 4, wherein the cellulose acylate constitutes at least a part of a surface of a member that comes into contact with blood.

6. Use according to any one of claims 1 to 5, wherein the filtering medium is a nonwoven fabric or a porous membrane.

7. Use according to any one of claims 1 to 6, wherein the cellulose acylate includes two or more acyl groups selected from the group consisting of an acetyl group, a propionyl group, and a butyryl group.

8. Use according to any one of claims 1 to 7, wherein the average through-hole diameter is 5 to 15 $\mu$m and the specific surface area is 3 to 50 m$^2$/g.

9. Use according to any one of claims 1 to 8, wherein the filtering medium is a nonwoven fabric made of fibers having an average fiber diameter of 1 nm to 5 $\mu$m and an average fiber length of 1 mm to 1 m, wherein the average fiber diameter and the average fiber length were determined by analyzing the electron microscopic observation image.

10. Use according to any one of claims 1 to 9, wherein the blood component is a blood cell component.

11. Use according to any one of claims 1 to 10, wherein the blood component is a leukocyte component.

12. A blood filter which is filled with the filtering medium for selective adsorption of blood components as defined in any one of claims 1 to 11.


**Patentansprüche**

1. Nicht-therapeutische Verwendung eines Filtermediums für die selektive Adsorption von Blutkomponenten, wobei das Filtermedium Celluloseacylat enthält, worin

die Glasübergangstemperatur des Filtermediums 126°C oder höher ist,
der mittlere Öffnungsdurchmesser 0,1 bis 50 $\mu$m beträgt und
die spezifische Oberfläche 1,0 bis 100 m$^2$/g beträgt, worin
die Glasübergangstemperatur und mittlere Öffnungsdurchmesser wie in der Beschreibung definiert bestimmt werden; und
die spezifische Oberfläche durch das Brunauer-Emmett-Teller-Verfahren mittels Stickstoffadsorption gemessen wird.

2. Verwendung gemäß Anspruch 1, worin der Substitutionsgrad einer Acylgruppe, die in dem Celluloseacylat enthalten ist, im Bereich von 1,00 bis 2,90 liegt, worin der Substitutionsgrad berechnet wird durch Vergleichen des Flächenintensitätsverhältnisses von Kohlenstoffen des Celluloseacylats, gemessen mittels eines $^{13}$C-NMR-Verfahrens.

3. Verwendung gemäß Anspruch 1 oder 2, worin in dem Celluloseacylat enthaltene Acylgruppe 4 oder weniger Kohlenstoffatome aufweist.

4. Verwendung gemäß irgendeinem der Ansprüche 1 bis 3, worin die in dem Celluloseacylat enthaltene Acylgruppe mindestens eine Acetylgruppe enthält.

5. Verwendung gemäß irgendeinem der Ansprüche 1 bis 4, worin das Celluloseacylat zumindest einen Teil einer Oberfläche eines Bauteils aufbaut, das in Kontakt mit Blut kommt.

6. Verwendung gemäß irgendeinem der Ansprüche 1 bis 5, worin das Filtermedium ein Vliesstoff oder eine poröse Membran ist.

7. Verwendung gemäß irgendeinem der Ansprüche 1 bis 6, worin das Celluloseacylat zwei oder mehr Acylgruppen enthält, ausgewählt aus der Gruppe bestehend aus einer Acetylgruppe, einer Propionylgruppe und einer Butyrylgruppe.

8. Verwendung gemäß irgendeinem der Ansprüche 1 bis 7, worin der mittlere Öffnungsdurchmesser 5 bis 15 $\mu$m beträgt und die spezifische Oberfläche 3 bis 50 m$^2$/g beträgt.

9. Verwendung gemäß irgendeinem der Ansprüche 1 bis 8, worin das Filtermedium ein Vliesstoff ist, hergestellt aus Fasern mit einem mittleren Faserdurchmesser von 1 nm bis 5 $\mu$m und einer mittleren Faserlänge von 1 mm bis 1 m, worin der mittlere Faserdurchmesser und die mittlere Faserlänge durch Analysieren eines Elektronenmikroskop-Beobachtungsbilds bestimmt werden.

10. Verwendung gemäß irgendeinem der Ansprüche 1 bis 9, worin die Blutkomponente eine Blutzellkomponente ist.

11. Verwendung gemäß irgendeinem der Ansprüche 1 bis 10, worin die Blutkomponente eine Leukocytenkomponente ist.

12. Blutfilter, der mit dem wie in irgendeinem der Ansprüche 1 bis 11 definierten Filtermedium für die selektive Adsorption von Blutkomponenten gefüllt ist.

**Revendications**

1. Utilisation non thérapeutique d'un milieu filtrant pour adsorption sélective de composants sanguins, le milieu filtrant contenant de l'acylate de cellulose, dans laquelle

   une température de transition vitreuse du milieu filtrant est de 126 °C ou plus,
   un diamètre moyen de trou traversant va de 0,1 à 50 $\mu$m, et
   une superficie spécifique va de 1,0 à 100 m$^2$/g, dans laquelle
   la température de transition vitreuse et le diamètre moyen de trou traversant sont déterminés comme défini dans la description ; et
   la superficie spécifique est mesurée par la méthode de Brunauer-Emmett-Teller par adsorption d'azote.

2. Utilisation selon la revendication 1, dans laquelle un degré de substitution d'un groupe acyle inclus dans l'acylate de cellulose est compris dans une plage allant de 1,00 à 2,90, dans laquelle le degré de substitution est calculé en comparant le rapport d'intensité de superficie de carbones d'acylate de cellulose mesuré par un procédé $^{13}$C-NMR.

3. Utilisation selon la revendication 1 ou revendication 2, dans laquelle le groupe acyle inclus dans l'acylate de cellulose présente 4 atomes de carbone ou moins.

4. Utilisation selon l'une quelconque des revendications 1 à 3, dans laquelle le groupe acyle inclus dans l'acylate de cellulose inclut au moins un groupe acétyle.

5. Utilisation selon l'une quelconque des revendications 1 à 4, dans laquelle l'acylate de cellulose constitue au moins une partie d'une surface d'un élément qui entre en contact avec le sang.

6. Utilisation selon l'une quelconque des revendications 1 à 5, dans laquelle le milieu filtrant est un tissu non tissé ou une membrane poreuse.

7. Utilisation selon l'une quelconque des revendications 1 à 6, dans laquelle l'acylate de cellulose inclut deux groupes acyle ou plus sélectionnés dans le groupe consistant en un groupe acétyle, un groupe propionyle et un groupe butyryle.

8. Utilisation selon l'une quelconque des revendications 1 à 7, dans laquelle le diamètre moyen de trou traversant va de 5 à 15 $\mu$m et la superficie spécifique va de 3 à 50 m$^2$/g.

9. Utilisation selon l'une quelconque des revendications 1 à 8, dans laquelle le milieu filtrant est un tissu non tissé composé de fibres présentant un diamètre moyen de fibre de 1 nm à 5 $\mu$m et une longueur moyenne de fibre de 1 mm à 1 m, dans laquelle le diamètre moyen de fibre et la longueur moyenne de fibre ont été déterminés en analysant l'image d'observation microscopique électronique.

10. Utilisation selon l'une quelconque des revendications 1 à 9, dans laquelle le composant sanguin est un composant de cellule sanguine.

11. Utilisation selon l'une quelconque des revendications 1 à 10, dans laquelle le composant sanguin est un leucocyte.

**12.** Filtre à sang qui est rempli du milieu filtrant pour adsorption sélective de composants sanguins tel que défini dans l'une quelconque des revendications 1 à 11.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3172542 B
- WO 2005120600 A
- JP 2016053232 A
- JP 2001198215 A
- JP H0624995 A
- JP 2012046843 A

- JP 11005851 A
- JP H11005851 A
- JP 2002212338 A
- JP 2002338601 A
- JP 5087808 A
- JP H05087808 A

**Non-patent literature cited in the description**

- Hatsumei Suishin Kyokai Kokai Giho (Journal of Technical Disclosure). Japan Institute for Promoting Invention and Innovation, 15 March 2001, 7-12

- Rikagaku Shoten (Dictionary of Physics and Chemistry. 2000